# EUROPEAN PATENT APPLICATION

(11) **EP 4 035 632 A1**
(43) Date of publication of application: **03.08.2022**
(21) Application number: 21183370.2
(22) Date of filing: 02.07.2021
(51) Int. Cl.: A61F 2/28, A61F 2/30

(54) **DEVICE FOR CRANIOPLASTY**

(30) Priority: 01.02.2021 IT 202100002066
(71) Applicant: H-Opera S.r.l., 84084 Fisciano (SA) (IT)
(72) Inventor: CATALDO, Emilio, 84091 BATTIPAGLIA (SA) (IT); NADDEO, Alessandro, 84121 SALERNO (SA) (IT); NADDEO, Francesco, 84131 SALERNO (SA) (IT); CAPPETTI, Nicola, 84090 MONTECORVINO PUGLIANO (SA) (IT)
(74) Representative: Conversano, Gabriele

(57) **Abstract**

Device for mesh deformation for the realization of bone prostheses, characterized in that it comprises a support (1) provided with a base plate (12), to which a plurality of vertical guides (11) are fastened, and a cage (3) comprising a first (31) and a second plate (32) provided with a plurality of holes configured to house said vertical guides (11) in sliding manner, so that said cage (3) can slide vertically and in parallel to said base plate (12), said first and second plate (31, 32) comprising also fastening means configured to fasten the one of the two plates to the other one removably, and being provided with a hollow central area (33), where said mesh to be deformed can be installed, and in that said base plate is configured to fasten a prototype (2) of the geometry to be obtained with said mesh, provided with a base integral thereto, so that the position of said prototype is wholly contained inside said hollow central area (33).

## Description

### Technical field

The present Patent application for industrial invention relates to a method for realizing prostheses needed in cranioplasty surgery.

### State of the art

There exist many pathological conditions, both of traumatic and degenerative nature, upon which bone lacunae appear in the human skull. In other situations, it is instead needed to remove a portion of the cranial vault, for example in case of surgery.

It is the case for example of traumatic conditions where, upon subdural hematomas, it is needed a serosanguinous drainage for which it is removed a portion of bone tissue of the skull.

Also, brain tumors can cause erosion of skull portions, thus leaving lacunae on the surface.

In these cases, it is essential to intervene surgically compensating the cranial lacuna by means of suitable prostheses in order to avoid that the patient can be subjected to further brain damage.

Many techniques exist to carry out this task.

At the beginning, lacunae were compensated autogenously, by depositing fragments of the same removed portion of the skull. Obviously, this technique was not possible when the portion of the skull was eroded due to degenerative pathologies, or if the portion of the skull was not usable for the aim, as in case of severe traumatic events.

To solve this problem, at the state of the art it is known the usage of fragments of custom-made cranial vaults (CAD projected and 3D printed with bio-compatible material) or of titanium structures interwoven to each other as a tissue, named "mesh". Such mesh structures are provided in "plate" shape (i.e. with planar surface) and in the following the surgeon has to deform them suitably, so to reproduce the geometry of the lacuna it is desired to fill.

The advantage of custom-made structures is that, theoretically, if well projected, they coincide perfectly with the cranial lacuna to be filled, thus giving back an optimized aesthetic effect in addition to a perfect functionality. Anyway, such structures require extremely long processing times (both for projecting and 3D printing realization), and these times are often incompatible with emergency surgery. Moreover, custom made cranial vaults are very expensive precisely because they are not standard structures, but they are projected and realized time by time for the specific patient.

Mesh structures, instead, are definitely cheaper and can be provided practically immediately, since they can be bought in stock; the material is bio-compatible as well and the structure is characterized by a specific texture which makes the integration with the rest of the bones easier. Anyway, since they are "standard" structures, it is the surgeon, who relies only on his own ability and manual skill, the one who has to reconstruct the surface of the lacking portion of bone tissue most closely, so that both an optimized aesthetic and functional effect is obtained.

Among the examples known at the state of the art, in US10842504 a method is described for optimizing the execution of cuts needed to give a bone fragment of a donor the suitable shape for the implant; in US20190192298 a method is described for the realization of cranioplasty prostheses in which the geometry is determined on the basis of diagnostic pre-surgery images; in WO2016086054 a method is described for the correct positioning in the space of a prosthesis for bone surgery.

### Technical problem

To the best of the current inventors' knowledge, there are not known devices favouring the realization of bone prostheses for cranial surgery and which allow to use standard prostheses and to customize them rapidly, reliably and iteratively.

### Aim of the invention

Aim of the present invention is to provide a device to favour the realization of bone prostheses for cranial surgery, which overcomes the limits linked to the embodiments known at the state of the art, and in particular which allows to use standard prostheses and to customize them rapidly, reliably and iteratively.

### Brief description

The invention realizes the prefixed aims since it is a device for mesh deformation for the realization of bone prostheses, characterized in that it comprises a support (1) provided with a base plate (12), to which a plurality of vertical guides (11) are fastened, and a cage (3) comprising a first (31) and a second plate (32) provided with a plurality of holes configured to house said vertical guides (11) in sliding manner, so that said cage (3) can slide vertically and in parallel to said base plate (12), said first and second plate (31, 32) comprising also fastening means configured to fasten the one of the two plates to the other one removably, and being provided with a hollow central area (33), where said mesh to be deformed can be installed, and in that said base plate is configured to fasten a prototype (2) of the geometry to be obtained with said mesh, said prototype being provided with a base integral thereto, so that the position of said prototype is wholly contained inside said hollow central area (33) .

### Description of the figures

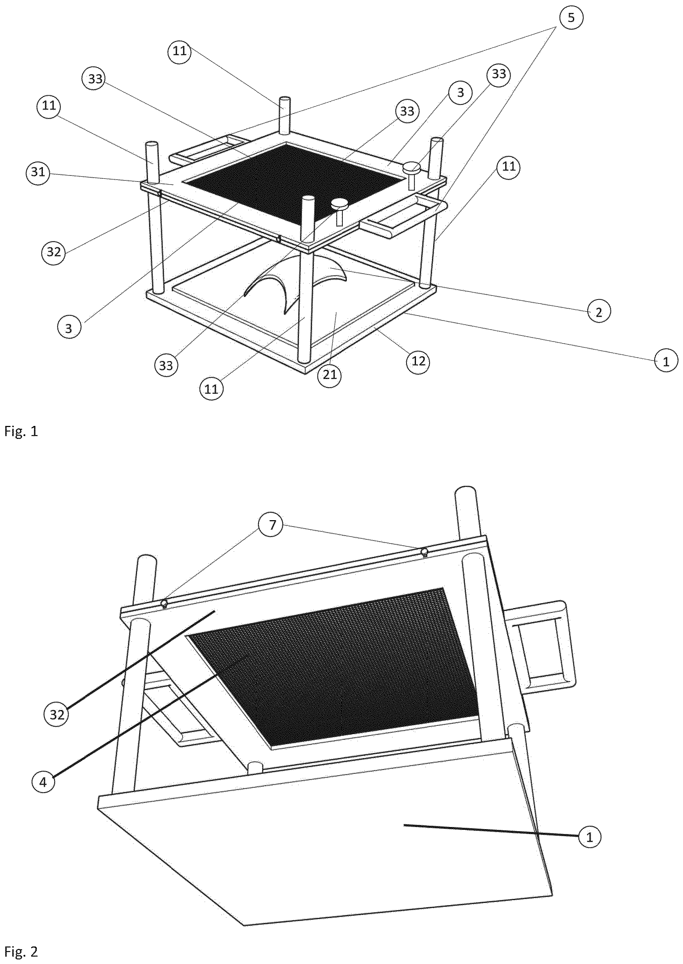

Figure 1 shows a top perspective view of the device to carry out the method according to the invention; figure 2 shows a bottom perspective view of the same device.

### Description of the invention

The object of the present invention is a device for the deformation of bone prostheses with the aim of combining the advantages of the two just described methods, i.e. the custom made projecting resulting precise and customized and the usage of standard materials which are easily available.

The essential idea of the present invention is to reproduce by CAD the geometry of the bone tissue which should fill the lacuna by means of custom made projecting, and to print it in 3D by FDM technologies with standard materials, not necessarily bio-compatible (in extremely cheaper and faster way than what happens with the sintering technologies needed for constructing custom made cranial vaults) and to use such model as "master" cast on which a standard reticular structure (mesh) is to be deformed in the following by using the device according to the invention.

The device is configured to make the mesh deformation process rapid and efficient.

With reference to the appended figures, the device comprises a base (1), on which the prototype (2) of the geometry to be reproduced is positioned.

It is to be specified that the geometry to be reproduced is preferably obtained by means of rapid prototyping starting from a CAD which reconstructs the geometry relating to the portion of cranial vault to be implanted.

According to what known per se at the state of the art, such geometry can be modelled by means of CAD tools starting from diagnostic images relating to the patient's skull. Said diagnostic images can comprise tridimensional tomography, images obtained by radiography or any other kind of diagnostic image apt to allow the tridimensional reconstruction of the portion of the skull object of surgery.

Moreover, the rapid prototyping of the geometry relating to the portion of cranial vault to be implanted is obtained by means of additive modelling techniques with plastic materials. These are techniques widely known at the state of the art. In particular, it is not needed that the material used for prototyping has particular biocompatibility features, since it will not be implanted.

The prototype (2) comprises conveniently - in addition to the reproduction of the geometry to be reproduced - a base (21) integral thereto and configured to be fastened stably to the support (1) of the device according to the invention.

In a preferred embodiment, said base (21) is parallelepiped-shaped and is provided with projections configured to engage respective holes provided on the support (1). Projections and holes coupling is not visible in the appended figures, in which the prototype (2) is shown yet assembled on the support (1).

Said base (21) is realized preferably by rapid prototyping together with the prototype (2).

In another embodiment, said base can be of standard type, can have fastening means for the support removable fastening, and the support can be realized with respective fastening means.

In this way, the base can be reused more times, and prototyping times are reduced.

The function of the base (21) is to keep the prototype (2) integral to the support (1) during the whole procedure described in the following.

The support (1) comprises a base plate (12) to which a plurality of vertical guides (1) are fastened, and a cage (3) comprising a first (31) and a second plate (32). Said plates (31, 32) comprise a plurality of holes configured to house said vertical guides (11) in a sliding manner, so that the cage (3) can slide vertically while remaining parallel to the base plate (12) of said support (1).

It is to be specified that, if needed as a function of the thickness of said plates, the upper plate (31) could comprise at said holes, configured to house said vertical guide (11) in a sliding manner, further guide elements, configured to reduce the alignment tolerances between the cage (3) and he base plate (12). For example, said guide elements could be tubes integral to said upper plate (31), configured to house said vertical guides (11) thereinside.

Said first (31) and second plate (32) comprise further a hollow centrale area (33), where a standard type mesh (4) can be installed. The mesh installation can occur by simply separating the two plates (31, 32), by resting the mesh (4) on the lower plate (32) and by overlapping the upper plate (31) thereto in the following.

Said hollow central area (33), or central hole, is such dimensioned that the mesh projects from the same from all sides, so that when the two plates overlap, the mesh (4) remains peripherally fastened therebetween.

Said plates (31, 32) comprise further fastening means configured to fasten removably the one of the two plates to the other one.

Preferably, but not limitingly, said fastening means are configured to vary the pressure exerted between the two plates, so that the sliding allowed between the mesh (4) upon tractive efforts is adjusted.

In a preferred embodiment, said fastening means comprise a plurality of knobs (33) comprising a screw and respective grasping means and configured to pass through through-holes provided in the upper plate, and to be screwed in respective threaded holes provided in the lower plate.

The pressure level of said plates in then determined by the greater or lower clamping of said knobs.

In another embodiment, said fastening means can comprise spring clamps, configured to clamp both said plates. The usage of spring clamps allows to have a known pressure between the two plates, independent of the manual clamping operation.

At least one of said plates comprise a couple of handles (5) configured to allow their easy moving. After describing all the components of the device, it is now possible to describe the usage.

After realizing a prototype (2) by rapid prototyping with the geometry of the skull portion for which a prosthesis has to be realized, the prototype can be fastened with its own base (21) to the base plate (12) of the support (1).

Now, the couple of holed plates (31, 32), overlapped between each other and with the mesh (4) installed according to what just described can be introduced in the sliding guides (11). By exerting a vertical thrust downwards by means of the handles (5), the mesh (4) is brought in contact with the prototype (2) and deforms itself thus taking the geometry defined by said prototype.

It is to be specified that said base (21) of the prototype is preferably dimensioned so that it cannot project with respect to said hollow central area (33) and has such a height that when said holed plates reach their end of stroke, the upper portion of said base (21) is higher than the upper face of the upper plate (31).

In this way, the mesh is necessarily subjected to such a deformation that it takes the shape of the upper surface of said prototype (2).

In this way, it is obtained the deformation of the mesh (4) along an arbitrary convex geometry, defined by the prototype (2).

Since, anyway, it could be needed that the mesh takes a concave geometry in some portions, the device comprises further preferably a further plate, not shown in the appended figures, configured to slide vertically along said vertical guides (11) and provided with fastening means for a second prototype.

Said second prototype will be realized according to the same modes as the first prototype (2) and will have a surface of perfectly overlapping shape to the one of said first prototype (2).

In other words, respective concave portions of the second prototype correspond to the convex portions of the first prototype, and respective convex portions of the first prototype correspond to the concave portions of the first prototype.

Moreover, also the second prototype is provided with a base integral thereto, configured to allow the fastening in a predetermined position to said further plate.

In this way, when the third plate with the second prototype fastened and directed downwards slides vertically along said vertical guides (11), the position of the first and second prototype are such that they coincide perfectly. A pressure of the third plate downwards obligates the mesh to be deformed also at the concave portions of the first prototype (2).

At the end of these operations, the deformed mesh can be removed from the two plates (31, 32) to be cut to length.

Therefore, as a result said mesh has taken the shape of an arbitrary surface, by using only the device according to the invention, standard prostheses and pieces realized by rapid prototyping, by means of widely diffused technologies by now. It is important to state again that there is no need to use particular materials for the realization of the prototypes, since the prototypes will never come in contact to the human body, and after being deformed the mesh can be sterilized and washed with techniques known per se at the state of the art.

## Claims

1. Device for mesh deformation for the realization of bone prostheses, **characterized in that** it comprises a support (1) provided with a base plate (12), to which a plurality of vertical guides (11) are fastened, and a cage (3) comprising a first (31) and a second plate (32) provided with a plurality of holes configured to house said vertical guides (11) in sliding manner, so that said cage (3) can slide vertically and in parallel to said base plate (12),
said first and second plate (31, 32) comprising also fastening means configured to fasten the one of the two plates to the other one removably, and being provided with a hollow central area (33), where said mesh to be deformed can be installed, and **in that**
said base plate is configured to fasten a prototype (2) of the geometry to be obtained with said mesh, said prototype being provided with a base integral thereto, so that the position of said prototype is wholly contained inside said hollow central area (33) .

2. Device according to claim 1, **characterized in that** the geometry of said prototype (2) in obtained by means of rapid prototyping of the geometry relating to the portion of cranial vault to be implanted.

3. Device according to claim 1 or 2, **characterized in that** said prototype (2) comprises a base (21) integral thereto and configured to be fastened stably to said support (1).

4. Device according to claim 3, **characterized in that** said base (21) is parallelepiped-shaped and is provided with projections configured to engage respective holes provided on the support (1).

5. Device according to any one of the preceding claims, **characterized in that** said upper plate (31) comprises tubes integral thereto, configured to house said vertical guides (11) thereinside.

6. Device according to any one of the preceding claims, **characterized in that** said hollow central area (33) is such dimensioned that the mesh projects from the same from all sides, so that when the two plates overlap, the mesh (4) remains peripherally fastened therebetween.

7. Device according to any one of the preceding claims, **characterized in that** said fastening means are configured to vary the pressure exerted between the two plates, so that a minimum sliding between the mesh (4) upon tractive efforts is allowed.

8. Device according to any one of the preceding claims, **characterized in that** said base (21) of the prototype is dimensioned so that it cannot project with respect to said hollow central area (33) and has such a height that when said holed plates reach their end of stroke, the upper portion of said base (21) is higher than the upper face of the upper plate (31).

9. Device according to any one of the preceding claims, further comprising a further plate configured to slide vertically along said vertical guides (11) and provided with fastening means for a second prototype, said second prototype having a surface of shape which can be overlapped to the one of said first prototype (2).
